# EUROPEAN PATENT APPLICATION

(11) **EP 0 824 096 A2**
(43) Date of publication of application: **18.02.1998**
(21) Application number: 97202331.1
(22) Date of filing: 29.07.1997
(51) Int. Cl.: C07C 67/14, C07C 69/653, C07D 213/68, C07D 213/64, C08F 220/22, G02B 6/00

(54) **Process for the preparation of halogenated (meth)acrylic esters and poly (meth) arcylates obtained with said (meth)acrylic esters**

(30) Priority: 13.08.1996 EP 96202276
(71) Applicant: Akzo Nobel N.V., 6824 BM Arnhem (NL)
(72) Inventor: Hofstraat, Johannes Willem, 7006 VG Doetinchem (NL); Wakselman, Claude, 75016 Paris (FR); Lequesne, Christelle, 75018 Paris (FR); Wiersum, Ulfert Elle, 6881 AV Velp (NL); Blazejewski, Jean Claude, 78650 Beynes (FR)
(74) Representative: Schalkwijk, Pieter Cornelis

(57) **Abstract**

The present invention is directed to a process for the preparation of (meth)acrylates of halogenated alcohols by direct esterification of said alcohols with (meth)acrylic acid (chloride) wherein at least 2,6-substituted pyridine derivative is used as a polymerization inhibitor at room temperature or lower. Suitable at least 2,6-substituted pyridine derivatives are 2,6-lutidine, 2,4,6-collidine or 2,6-di-tert.butyl-4-methyl pyridine.

With the halogenated (meth)acrylate esters prepared with the process according to the invention, very pure homo- and copolymers can be prepared, resulting in polymers having a low optical loss of 0.1 dB/cm or lower at 1300 nm and lower than 0.4 dB/cm at 1550 nm.

## Description

The present invention is directed to a process for the preparation of (meth)acrylates of halogenated alcohols by direct esterification of said alcohols with (meth)acrylic acid/derivatives and polyacrylates obtained by polymerization of said acrylic esters. The (meth)acrylates of halogenated alcohols, which exhibit a high degree of purity, are particularly suitable for the production of polymers and copolymers to give highly transparent polymeric products. The refractive index of these polymeric products can easily be set by varying the amount of halogen and/or the ratio of the applied combination of halogen (e.g. the fluorine/chlorine ratio). This is required for optical applications such as waveguiding structures. In consideration of the high cost of halogenated alcohols it is essential to have available a method for the preparation of (meth)acrylates which does not require an excess of alcohol and which, besides providing high yields, ensures first and foremost that an easily purifiable ester is obtained.

The formation of acrylic-type esters from alcohols and the corresponding acid chlorides is performed in the presence of a base.
Usually, this base is a tertiary amine or pyridine. For example, allylic esters of trifluoromethacrylic acid have been prepared in the presence of triethyl amine, as is described in J. Org. Chem., 56 (1991), 1718, and pentafluoroaryl methacrylates have been formed in pyridine medium, as is described in J. Mater. Chem.,3 (1993), 15. In EP-A1-0 160 379 tetrafluorophenol is esterified with methacrylic acid chloride in the presence of a hydrogen chloride trapping agent such as alkali metal hydroxide, magnesium carbonate, pyridine, and molecular sieves. In EP-A1-0 230 656 1-phenyl-2,2,2-trifluorethyl-α- chloroacetate is prepared by esterification of 1-phenyl-2,2,2-tri fluoroethanol with acrylic acid chloride in the presence of pyridine or quinoline. In US 344,535 the esterification of 2-(pentafluorophenyl)-hexafluoroisopropanol with acrylic acid chloride is described. The presence of trifluoric anhydride, pyridine, quinoline, triethylamine, and N,N'-dimethyl aniline is recommended In the case of halogenated phenyl acrylates, extensive polymerization occurs when conventional bases are used. In US 3,845,102 a process for the preparation of methacrylic esters of multi-brominated phenol derivatives have been disclosed in the presence of a pyridine base, particularly pyridine, α-picoline, and β-picoline. Reaction temperatures are preferably in the range of 100°C to 160°C, but the reaction proceeds too slowely at temperatures below 50°C. Because of these high reaction temperatures a non-pyridine polymerization inhibitor such as cuprous chloride is usually necessary. These reaction conditions are disadvantageous because polymerization is a serious side-reaction at higher temperatures. Particularly, acrylic acid derivatives cannot be submitted to such reaction conditions. It is therefore an object of the invention to find mild reaction conditions for performing the esterification reaction with minimal polymerization of the (meth)acrylate derivative. With the process according to the invention this unwanted polymerization reaction is greatly limited.

To this end the present invention is directed to a process for the preparation of (meth)acrylates of halogenated alcohols by direct esterification of said alcohols with (meth)acrylic acid derivative wherein at least 2,6-substituted pyridine derivative is used as a polymerisation inhibiting catalyst at 50°C or lower. Preferably the reaction is performed at room temperature or lower.

It was found that the objectionable polymerization reaction is repressed by the use of a pyridine derivative which is less nucleophilic and more sterically hindered than pyridine.

Within the context of this description (meth)acrylic acid derivative means acid chlorides, acids, and esters.

Halogenated acrylic esters which are highly suitable for use in optical polymers or copolymers contain the lowest possible amount of aliphatic C-H bonds, notably CH₃ groups, per volume unit. This is to reduce optical losses in the wavelength ranges generally used in the optical field (600-700 nm, 780-980 nm, and especially the near-infrared region of 1200-1600 nm). An additional advantage of the use of halogenated acrylic esters and their polymers is their resistance to singlet oxygen.
Suitable starting materials for obtaining these acrylic esters are the halogenated alcohols from any one of formulae 1-15: 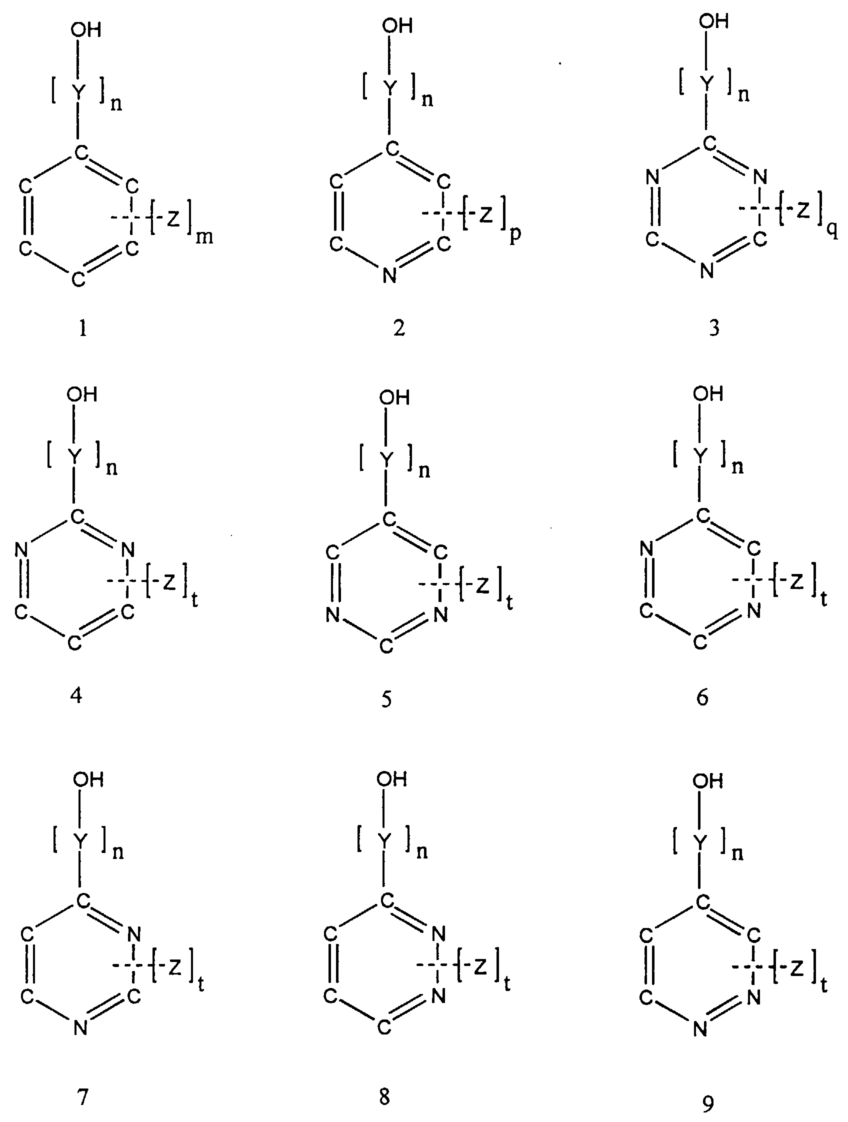 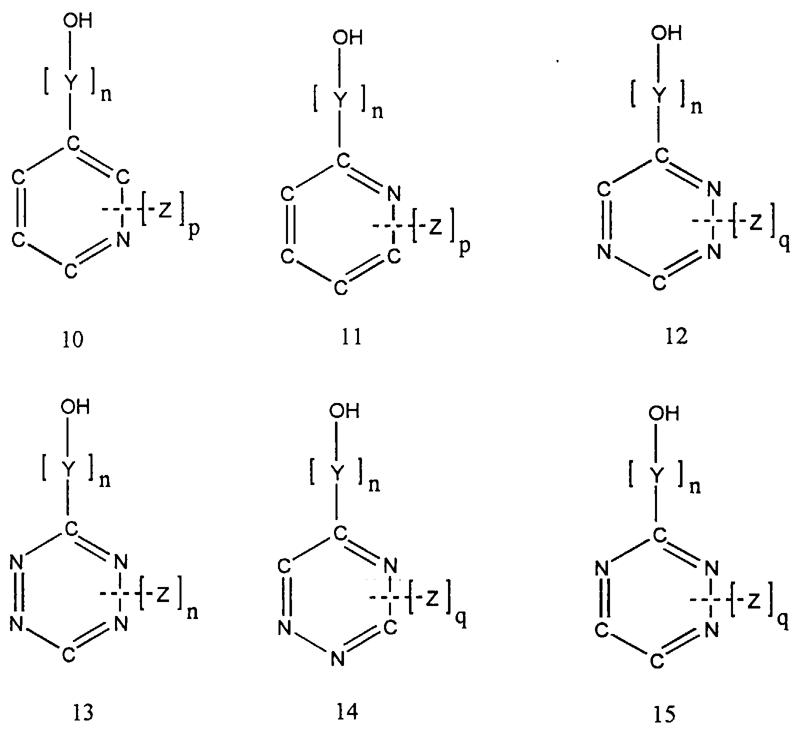 wherein
- Y: stands for -C(Chal₃)₂-,
- n: stands for 1 or 0
- hal: stands for halogen,
- Z: stands for -F, -Cl, -D, -Br, -CF₃
- m: is an integer 1-5, wherein the Z atoms can be chosen independently from each other,
- p: is an integer 1-4, wherein the Z atoms can be chosen independently from each other,
- q: is an integer 1-2, wherein the Z atoms can be chosen independently from each other,
- t: is an integer 1-3, wherein the Z atoms can be chosen independently from each other.

Fully halogenated alcohols are preferred because also aromatic C-H bond influence the light loss detrimentally.

Suitable (meth)acrylic acid derivatives are 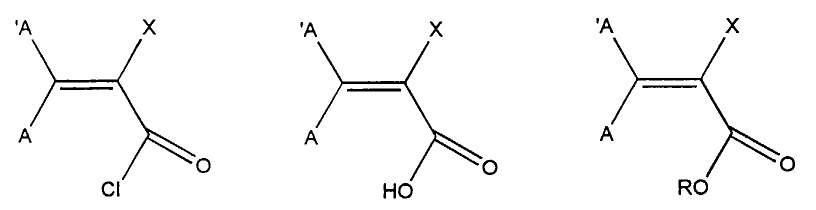 wherein
X stands for -H, -CH₃, -F, -Cl, -OCF₃, -OCCl₂F, -OCClF₂, -SCF₃, -SCCl₂F, -SCClF₂, a wholly or partially halogenated and/or deuterated linear alkyl group having 1-6 carbon atoms, a wholly or partially halogenated branched alkyl group having 1-6 carbon atoms,
A stands for -H, -D, or -hal,
A 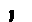 is independently chosen from A and may be the same groups as A,
R stands for an alkyl group having 1-6 carbon atoms.

As mentioned above, it is thought essential that the base is less nucleophilic and more sterically hindered than pyridine. Suitable at least 2,6-substituted pyridine derivatives are 2,6 dialkylpyridine derivatives such as 2,6-lutidine, 2,4,6-collidine, 2,6-di-tert.butylpyridine, or 2,6-di-tert.butyl-4-methyl pyridine.

(Meth)acrylic esters obtained through the esterification of alcohols according to formulae 2-15 given above and (meth)acrylic acids have not been described hereinbefore. The present invention is also directed to these novel (meth)acrylic esters. The homo- and copolymers prepared by polymerization of said (meth)acrylic esters appear to have higher Tgs than the benzene ring-containing acrylic esters. The present invention is also directed to homo-and copolymers prepared by polymerization of said acrylic esters.

As mentioned above, for optical applications it is required that the refractive index of the polymer material can be easily set. The refractive index of the resulting polymers can be set very accurately. With the halogenated (meth)acrylate esters prepared with the process according to the invention, very pure homo- and copolymers can be prepared, resulting in polymers having a low optical loss of 0.1 dB/cm or lower at 1300 nm and lower than 0.4 dB/cm at 1550 nm. Further, the maximum light loss within the optical window of 1200-1600 nm is 1.5 dB/cm. This means that, in contrast to PMMA, the polymers according to the invention can suitably be used for waveguides within the whole optical window of 1200-1600 nm. Homo- and copolymers having these advantageous properties have not been obtained so far and the invention is also directed to these polymers.

The index of refraction of copolymers of pentafluorophenyl (meth)acrylate and tetrachloroethyl acrylate can be set accurately to make a core material for optical waveguides with (cheap) PMMA claddings. Especially, copolymers built from a monomer mixture comprising 45-65 wt% tetrachloroethyl acrylate and 35-55 wt% pentafluorophenyl acrylate are preferred for their suitable index of refraction. Even more preferred are copolymers built from said monomer mixture also comprising upto 10 wt% non-halogenated cross-linker such as ethylene glycol di(meth)acrylate (EGD(M)A), butanediol diacrylate (BDA) and, triallylcyanurate (TAC) or upto 30 wt% halogenated cross-linker. Also copolymers built from a monomer mixture comprising 45-60 wt% pentafluorophenyl methacrylate, 50-35 wt% tetrachloroethyl acrylate and upto 10 wt% non-halogenated cross-linker appear to have a suitable index of refraction to make a core material for optical waveguides with PMMA claddings.

The polymerization reaction to form the monomers into polymers can be performed with either thermal initiators or photoinitiators. The various embodiments of the polymerization reaction (emulsion polymerization, suspension polymerization, solution polymerization, and bulk polymerization) are known to the skilled person. For a description of these reactions reference may be had to, int. al., FR-2 623 510, and they require no further elucidation here. In the case of bulk polymerization being performed, it is recommended to carry out a postcuring at elevated temperature after the initial curing with a photoinitiator or thermal initiator. This leads to improved thermal resistance and reduced optical loss.

Polymeric optical waveguiding structures generally comprise a polymeric-three layer structure on a substrate. The three layer structure comprises a low refractive index lower cladding layer, a high refractive index core layer, and a low refractive upper cladding layer. The polymeric optical devices may also comprise more layers such as a five layer structure as is described in European patent application No. 95201762.2.

The polymers according to the invention are pre-eminently suited to be used as core material in waveguiding structures, but they may also be used as waveguiding structure cladding. Since the index of refraction of these polymers is easy to set, it is preferred to make both the core material and the cladding of waveguiding structures of polymers according to the invention. In that case the refractive indices of the core and the cladding can be matched, while the other properties (thermal coefficient of expansion, Tg, the refractive index's dependence on temperature) remain virtually the same. An additional advantage of the use of polymers according to the invention for both core material and cladding consists in that the optical loss is reduced further still.

The above is of advantage especially when preparing waveguiding structures according to the methods described in EP-0 560 043, DE-4231113, DE-4220135, DE-4423112, and WO 93/21550, which involve using a mould to make a moulded article of polymer material which serves as cladding. The moulded article has recesses for coupling in optical fibres and for core material. The polymers according to the invention are particularly suited to be used for either moulding material or core material.

The polymers according to the invention may be built up from a monomer mixture comprising halogenated acrylic esters prepared by the process according to the invention, styrenes, allyl ethers, and vinyl ethers. Preference is given to polymers of at least 50 wt.% is made up of acrylic esters according to the invention, since they have a high glass transition temperature (Tg). Polymers with a high Tg are desired, since they give waveguiding structures with a temperature resistance superior to that of structures made of low Tg polymers.

The use of halogenated alcohols according to formulae 1-15 having Y groups is preferred, because they result in polymers with an especially low optical loss.

Also displaying a low optical loss and being preferred are copolymers of halogenated acrylic esters according to the invention and substituted maleimides with a halogenated phenyl group on the nitrogen atom (see formula below). 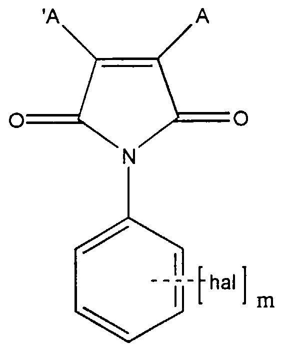 wherein A stands for -H, -D, -Cl, -Br, or an alkyl having 1 or 2 carbon atoms.

When processing the obtained polymers it is sometimes useful to have cross-linkable polymers, for instance, when layer-on-layer spincoating is envisaged. Cross-linking can be achieved by adding non-halogenated cross-linkers such as diacrylates (e.g. ethylene glycol di(meth)acrylate and butane diol acrylate) or triallyl cyanurate or triallyl isocyanurate to the monomer mixture. Halogenated cross-linkers such as halogenated acrylic esters prepared by the process according to the invention may be used as well. Generally, diacrylates are obtained by trans-esterifying diols. Particularly suitable, in view of the desired low optical loss in the near-infrared region, are diacrylates obtained from diols according to the general formulae below: 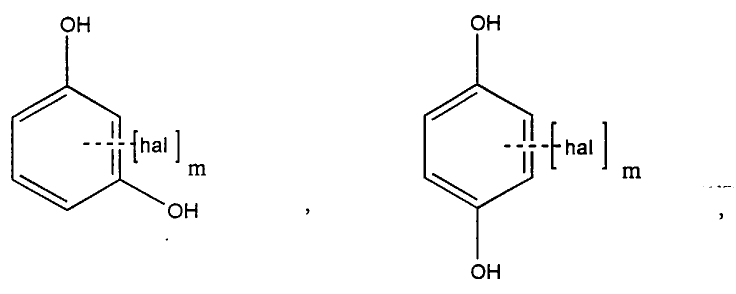 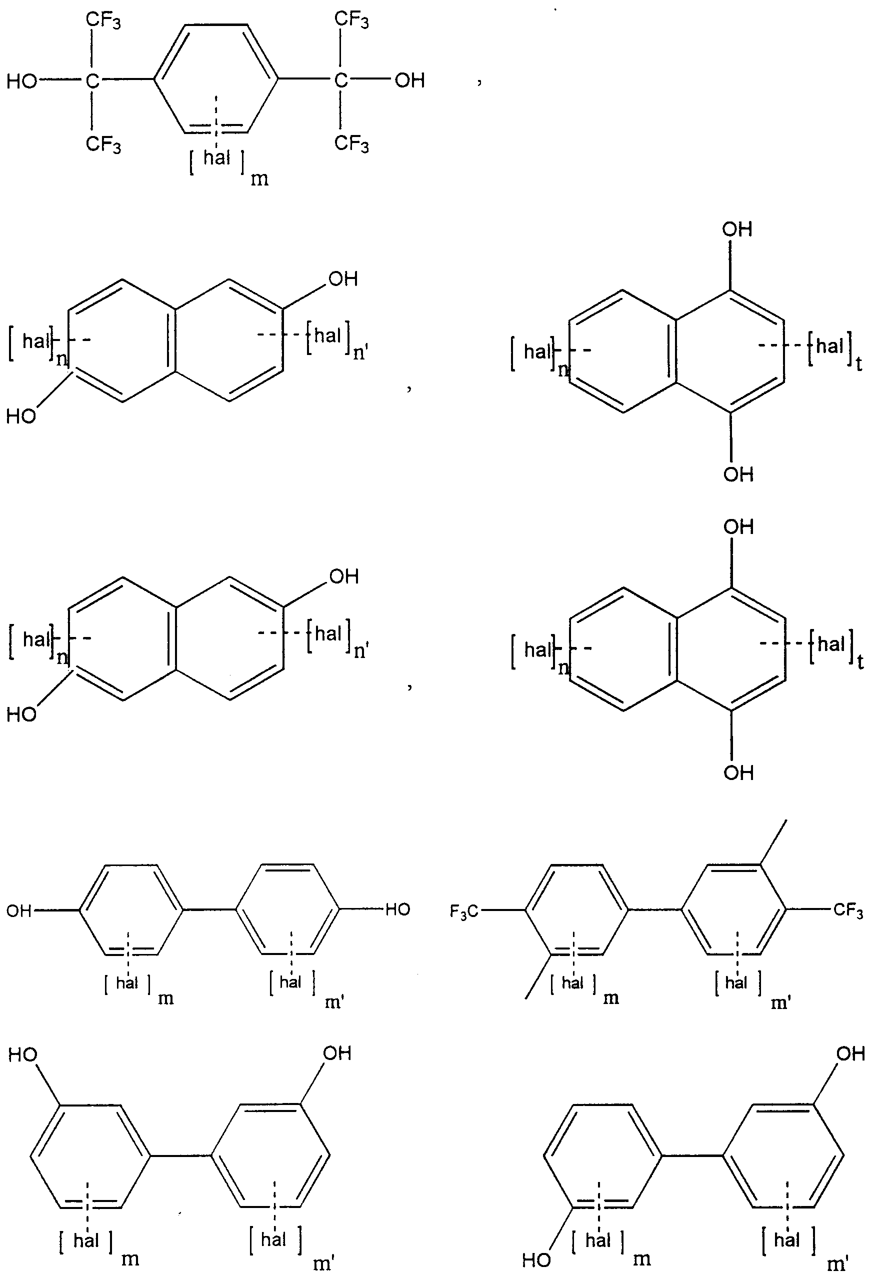 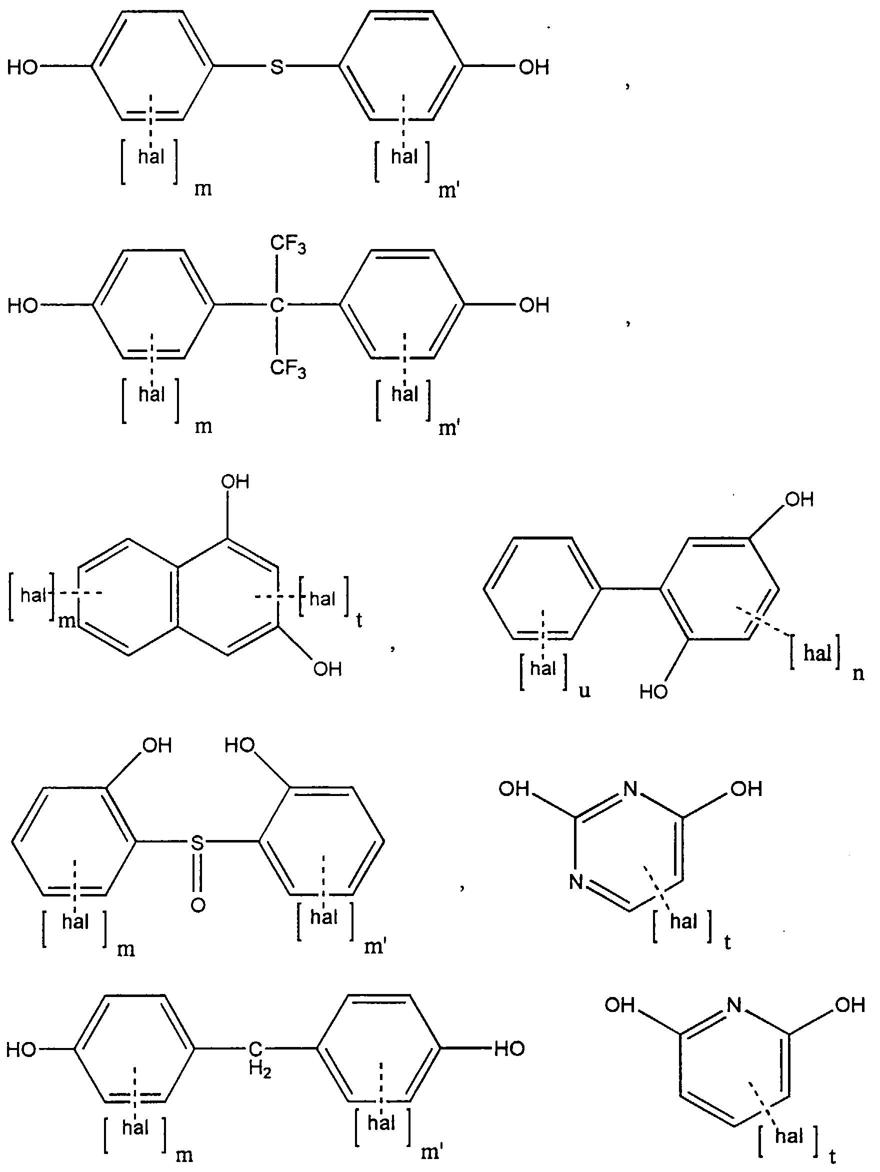 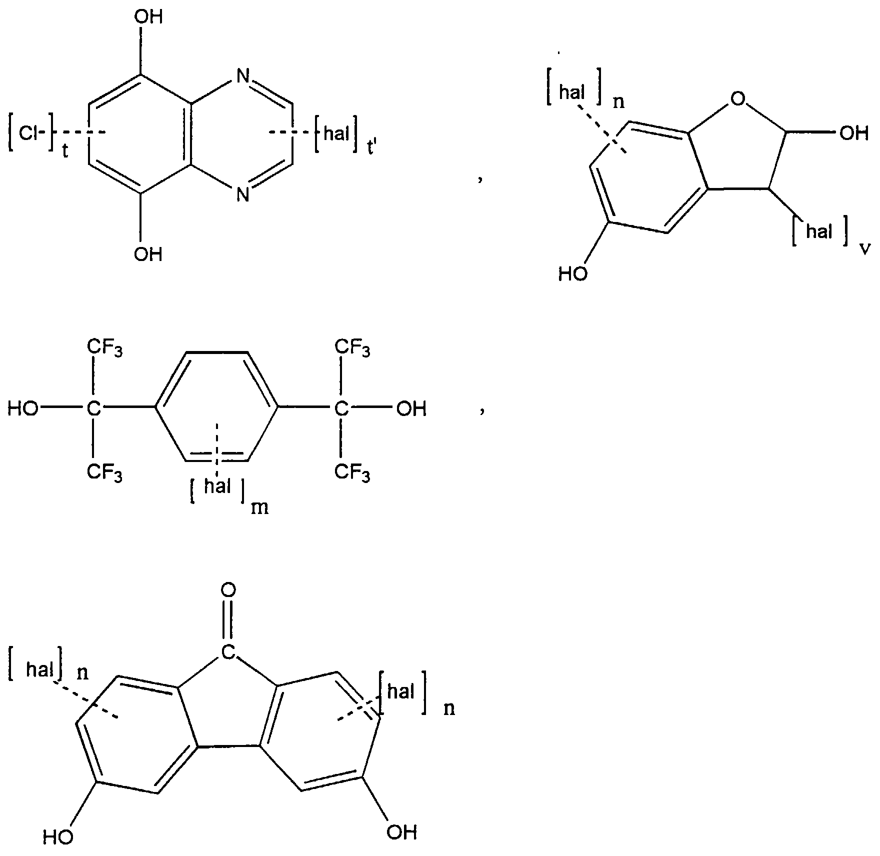 wherein
- n: as well as n 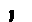 is: 1-3
- m: as well as m 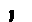 is: 1-4
- u: is: 1-5,
- t: is: 1-2, and
- v: is: 1 or 0.

Particularly suitable to be used are,
2,4-dichloro-1,3-benzenediol,
2,4,6-trichloro-1,3-benzenediol,
2,4,5-trichloro-1,3-benzenediol,
2,5-dichloro-1,3-benzenediol,
4,5-dichloro-1,3-benzenediol,
4,5,6-trichloro-1,3-benzenediol,
2,4,5,6-tetrachloro-1,3-benzenediol,
2,4,5,6-tetrafluoro-1,3-benzenediol,
2,4,5-trifluoro-1,3-benzenediol,
4,6-dibromo-1,3-benzenediol,
2,4,6-tribromo-1,3-benzenediol,
2,4-dibromo-1,3-benzenediol,
2-bromo-4-chloro-1,3-benzenediol,
4-bromo-6-chloro-1,3-benzenediol,
2-bromo-4,6-dichloro-1,3-benzenediol,
2,4-bromo-6-chloro-1,3-benzenediol,
2,3-dichloro-1,4-benzenediol,
2,6-dichloro-1,4-benzenediol,
3,6-dichloro-1,4-benzenediol,
2,5-dichloro-1,4-benzenediol,
2,3,5,6-tetrachloro-1,4-benzenediol,
2,3,5-trichloro-1,4-benzenediol,
2,3-difluoro-1,4-benzenediol,
2,6-difluoro-1,4-benzenediol,
3,6-difluoro-1,4-benzenediol
2,5-difluoro-1,4-benzenediol,
2,3,5,6-tetrafluoro-1,4-benzenediol,
2,3,5-trifluoro-1,4-benzenediol,
2,3-dibromo-1,4-benzenediol,
2,6-dibromo-1,4-benzenediol,
3,6-dibromo-1,4-benzenediol,
2,5-dibromo-1,4-benzenediol,
2,3,5,6,-tetrabromo-1,4-benzenediol,
2,3,5-tribromo-1,4-benzenediol,
2,3,5-tribromo-6-chloro-1,4-benzenediol,
2,3,5,6-tetrafluoro-α,α,α 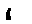 ,α 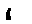 -tetrakis(trifluoromethyl)-1,4-benzene dimethanol,
3,6-dibromo-1,8-dichloro 2,7-naphthalenediol,
1,3,6,8-tetrabromo 2,7-naphthalenediol,
1,3,6-tribromo-8-chloro 2,7-naphthalenediol,
1,3,5,7-tetrabromo 2,6-naphthalenediol,
2,4,6,8-tetrachloro 1,5-naphthalenediol,
2,4,5,6,7,8-hexafluoro-1,3-naphthalenediol,
2,3,5,6,7,8-hexafluoro-1,4-naphthalenediol,
3,3',5,5'-tetrachloro [1,1'-biphenyl]-4,4'-diol,
2,2',3,3',5,5',6,6'-octachloro [1,1'-biphenyl]-4,4'-diol,
2,2',3,3',5,5',6,6'-octafluoro [1,1'-biphenyl]-4,4'-diol,
2,2',3,3',5,5',6,6'-octabromo [1,1'-biphenyl]-4,4'-diol,
2,2'5,5',6,6'-hexafluoro-4,4'-bis(trifluoromethyl)-[1,1'-biphenyl]-3,3'-diol,
2',3,3',4,4',5',6,6'-octafluoro-2,5-biphenyldiol,
3,3',4,4',6,6'-hexachloro-2,2'-thiobisphenol,
2,2',3,3',5,5',6,6'-octbromo-4,4'-thiobisphenol,
3,3',4,4',6,6'-hexachloro-2,2'-sulfinyl bisphenol,
3,3',4,4',6,6'-hexafluoro-2,2'-sulfinyl bisphenol,
3,3',4,4',6,6'-hexabromo-2,2'-sulfinyl bisphenol,
2,2'3,3'5,5'6,6'-octafluoro,chloro,bromo hexafluoro-bisphenol A,
2,3,3',5,5',6-hexafluoro,chloro,bromo hexafluoro-bisphenol A,
3,3',5,5'-tetrafluoro,chloro,bromo hexafluoro-bisphenol A,
2,2',3,3',5,5',6,6'-octafluoro 4,4'-methylene-bisphenol A,
2,2',3,3',5,5',6,6'-octachloro 4,4'-methylene-bisphenol A,
2,2',3,3',5,5',6,6'-octabromo 4,4'-methylene-bisphenol A,
2,3,3',5,5',6-hexafluoro,chloro,bromo 4,4'-methylene-bisphenol A,
3,3',5,5'-tetrafluoro,chloro,bromo 4,4'-methylene-bisphenol A,
6-chloro-2,4-pyrimidinediol,
6-bromo-2,4-pyrimidinediol,
6-fluoro-2,4-pyrimidinediol,
5,6-dichloro-2,4-pyrimidinediol,
5,6-dibromo-2,4-pyrimidinediol,
5,6-difluoro-2,4-pyrimidinediol,
2,3-dichloro-5,8-quinonediol,
2,3-difluoro-5,8-quinonediol,
2,3-dibromo-5,8-quinonediol,
2,3,6,7-tetrachloro-5,8-quinone iol,
2,3,6,7-tetrafluoro-5,8-quinone diol,
2,3,6,7-tetrabromo-5,8-quinone diol,
3,4,6,7-tetrachloro-2,3-dihydro-2,5-benzofuran diol,
3,4,6,7-tetrafluoro-2,3-dihydro-2,5-benzofuran diol,
3,4,6,7-tetrabromo-2,3-dihydro-2,5-benzofuran diol,
2,4,5,7-tetrachloro-3,6-dihydroxy-xanthen-9-one,
2,4,5,7-tetrafluoro-3,6-dihydroxy-xanthen-9-one,
2,4,5,7-tetrabromo-3,6-dihydroxy-xanthen-9-one,
   as well as aliphatic, halogenated diols according to the formulae below.

HOCH₂(CF₂CFCl)CH₂CH₂OH

HOCH₂(CF₂CFCl)₂CH₂CH₂OH

HOCH₂CH(SC₂H₄C₄F₉)CH₂CH₂OH

HOCH₂CH(SC₂H₄C₆F₁₃)CH₂CH₂OH

HOCH₂C(CH₂CH₃)(CH₂OC₃H₆SC₂H₄C₄F₉)CH₂OH

HOCH₂C(CH₂CH₃)(CH₂OC₃H₆SC₂H₄C₆F₁₃)CH₂OH

HOCH₂CH(CH₂C₄F₉)CH(CH₂C₄F₉)CH₂OH

HOCH₂CH(CH₂C₆F₁₃)CH(CH₂C₆F₁₃)CH₂OH

HOCH₂CH(CH₂C₈F₁₇)CH(CH₂C₈F₁₇)CH₂OH

HOCH₂C(CH₂SC₂H₄C₄F₉)₂CH₂OH

HOCH₂C(CH₂SC₂H₄C₆F₁₃)₂CH₂OH

HOCH₂C(CH₂SC₂H₄C₈F₁₇)₂CH₂OH

The diacrylates listed above are halogenated and so do not give any extra loss of light. The use of cross-linked polymers for spincoating reduces the risk of the subjacent layer dissolving again when a new layer of polymer is applied. Another advantage of cross-linked polymers consists in their increased mechanical stability and higher Tg.

### EXAMPLES

### Example 1

### Preparation of pentafluorophenyl acrylate (acrylic ester 1, AE1)

Acryloyl chloride (14.8 g, 164 mmoles) was added dropwise (40 min.) to a stirred solution of pentafluorophenol (15.1 g, 82 mmoles), 2,6-lutidine (13.2 g, 123 mmoles), and a catalytic amount of DMAP (4-dimethylaminopyridine) in THF (200 ml), which was cooled to 0°C. At the end of the addition, the precipitated lutidine hydrochloride was filtered and washed with pentane. The combined filtrates were concentrated by rotary evaporation under water aspirator pressure. The residual oil was purified by elution with pentane through Florisil® (60-100 mesh grade), yielding 16.7 g (86%) of acrylate ester 1 as a colorless oil.

### Example 2

### Preparation of pentafluorophenyl methacrylate (acrylic ester 2, AE2)

Acrylic ester 2 was prepared in the same way as acrylic ester 1 using methacryloylchloride.

### Example 3

### Reaction of trifluoromethacrylic acid with phthaloyl chloride

A mixture of trifluoromethacrylic acid (8 g, 57.1 mmoles) and phthaloyl chloride (17.4 g, 85.7 mmoles) was heated at 110°C for 4 hours. After cooling to room temperature, the crude mixture was subjected to short-path distillation (50°C, 0.07 mm Hg), yielding a 3:2 mixture of acid chlorides 1 and 2 (7g):

### Preparation of pentafluorophenyl trifluoromethacrylate (methacrylic ester 3, AE3)

To a stirred solution of pentafluorophenol (6.4 g, 34.8 mmoles) in pentane (150 ml) at 0°C, containing a catalytic amount of DMAP and a few crystals of hydroquinone, was added 2,6-lutidine (4.5 g, 42 mmoles). After stirring for 10 minutes at 0 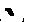 C, a mixture of acid chlorides 1 and 2 (4.3 g, 24.8 mmoles) was added dropwise. The reaction was stirred for an additional 15 minutes at 0°C and then filtered immediately, the receiver being cooled to 0°C. The precipitate was washed twice with cold (0°C) pentane. Immediate purification of the combined filtrates through Florisil® (60-100 mesh grade) using pentane as eluant yielded methacrylic ester 2 as a colorless oil (5.4 g 71%).

### Example 4

### Preparation of 2-(pentafluorophenyl)hexafluoropropan-2-ol

A solution of n-butyl lithium in hexane (1.6 M, 51 ml, 81.6 mmoles) was added dropwise in 40 minutes to a cold (-55°C) stirred solution of chloropentafluorobenzene (15 g, 74.1 mmoles) in diethyl ether (150 ml) under an argon atmosphere. The mixture was stirred at -55°C for 4 hours. A stream of hexafluoroacetone (30 g, 180 mmoles) was then passed over the cold solution for 30 minutes. The solution was allowed to warm up to 10°C and hydrolyzed with 1N aqueous hydrochloric acid (90 ml). The organic layer was removed and the remaining aqueous phase was extracted twice with ether (2X30 ml). The combined organic layers were dried over magnesium sulfate and concentrated under reduced pressure. Distillation of the residue under vacuum (56°C, 10 mm Hg) yielded 17.7 g (72%) of the fluorinated alcohol as a colorless oil.

### Preparation of 2-(pentafluorophenyl)hexafluoroprop-2-yl acrylate (acrylic ester 4, AE4)

2,6-Lutidine (4.3 g, 40.3 mmoles) was added at room temperature to a stirred solution of the alcohol prepared above (9 g, 26.9 mmoles) in pentane (100 ml) containing a catalytic amount of DMAP. After stirring for 20 minutes, acryloyl chloride (12.1 g, 134.2 mmoles) was rapidly added dropwise. After stirring for 19 hours, the precipitate was filtrated and washed twice with pentane. After concentration of the organic phase, the residue was purified by elution through Florisil® using pentane as eluant. Short-path distillation of the filtrate (80°C, 0.1 mm Hg) yielded 6.9 g (66%) of acrylic ester 4 as a colorless oil which slowly solidified at room temperature.

### Example 5

### 4-Hydroxy-tetrafluoropyridine

A mixture of pentafluoropyridine (40 g, 0.24 mole) and potassium hydroxide 28 g, 0.49 mole) in water (400 ml) was heated at 85°C during 20 h. After cooling, the aqueous phase was washed with dichloromethane (3x100 ml), acidified with 5N hydrochloric acid, and extracted with diethyl ether (3x100 ml). After drying of the ethereal phase over MgSO₄, the solvent was removed under reduced pressure to leave a semi-solid. Fractional sublimation (80°C, 2-3 mm Hg) yielded 65% (26 g) 4-hydroxy-tetrafluoropyridine as a white solid (m.p. 97°C).

### Tetrafluoropyrid-4-yl acrylate (acrylic ester 5, AE5)

A solution of 4-hydroxy-tetrafluoropyridine (10 g, 60 mmoles), 2,6-lutidine (9.7 g, 90 mmoles) and DMAP (catalytic) in THF (80 ml), was stirred at 0°C for half an hour. Freshly distilled acryloyl chloride (13.6 g, 150 mmoles) was added then dropwise (15 min). The mixture was stirred at 0°C for additional 45 min. The precipitated lutidine hydrochloride was filtered and washed twice with THF. Combined filtrates were concentrated by rotatory evaporation under water aspirator pressure. The residual oil was purified by elution with pentane/dichloromethane (80/20) through Florisil®, yielding 10.9 g (82%) of the tetrafluoropyridyl acrylate as a colorless oil.

### Example 6 and 7

### 3,5-Dichloro-2,6-difluoro-4-hydroxypyridine and 3,5-dichloro-4,6-difluoro/hyphen-2-hydroxypyridine

A mixture of 3,5-dichloro-2,4,6-trifluoropyridine (40 g, 0.2 mole) and potassium hydroxide (22.4 g, 0.4 mole) in water (400 ml) was heated at 85°C for 20 h. The reaction medium was cooled to room temperature and the aqueous phase was washed with dichloromethane (3x100 ml), acidified with 5N hydrochloric acid, and extracted with diethylether (3x100 ml). After drying of the ethereal phase over MgSO₄, the solvent was removed under reduced pressure to leave 37.9 g (95%) of a mixture of 4-hydroxy (67%) and 2-hydroxypyridine (33%).

### Example 8 and 9

### 3,5-Dichloro-2,6-difluoropyrid-4-yl acrylate and 3,5-dichloro-4,6-difluoropyrid-2-yl acrylate (acrylic esters 8 and 9, AE8, AE9)

2,6-Lutidine (2.9 g, 27 mmol) was added dropwise to a solution of 3,5-dichloro-2,6-difluoro-4-hydroxypyridine and 3,5-dichloro-4,6-difluoro-2-hydroxypyridine (63/37, 3.6 g, 18.0 mmole) containing DMAP (catalytic) in diethyl ether (50 ml). After stirring at room temperature for 15 min, freshly distilled acryloyl chloride (3.2 g, 35.7 mmole) was added dropwise (10 min). The mixture was vigorously stirred for 1 additional hour. The precipitated lutidine hydrochloride was filtered and washed twice with diethyl ether. Combined filtrates were concentrated by rotatory evaporation under water aspirator pressure (all removals of solvents were done at 0°C to minimize losses by sublimation). Further purification by elution with pentane/dichloromethane (80/20) through Florisil®, yielded a mixture of the 3,5-dichloro-2,6-difluoropyrid-4-yl acrylate as a white solid and 3,5-dichloro-4,6-difluoro pyrid-2-yl acrylate as a colorless oil.

### Examples 10-13

### 3-Chloro-4-hydroxytrifluoropyridine, 5-chloro-2-hydroxytrifluoropyridine, 3-chloro-2-hydroxytrifluoropyridine, and 4-chloro-2-hydroxytrifluoropyridine

A mixture of 3-chlorotetrafluoropyridine and 4-chlorotetrafluoropyridine (89/11, 20 g, 0.1 mole) together with potassium hydroxide (13.5 g, 0.2 mol) in water (200 ml) was heated at 85°C for 20 h. The reaction medium was allowed to cool to room temperature and the aqueous phase was washed with dichloromethane (3x50 ml), acidified with 5N hydrochloric acid, and extracted with diethylether (3x50 ml).The ethereal phases were pooled, washed with brine (1x50ml), and dried over MgSO₄. The solvent was removed under reduced pressure to leave a mixture of chlorotrifluoropyridinols. The ¹⁹F-NMR spectrum showed the composition of this mixture to be 3-chloro-4-hydroxytrifluoropyridine (74%), 5-chloro-2-hydroxytrifluoropyridin (13%), 3-chloro-2-hydroxytrifluoropyridine, (3%), and 4-chloro-2-hydroxytrifluoropyridine (10%). The mixture was purified by fractional sublimation (80°C, 2-3 mm Hg). The less volatile fraction was the pure 4-pyridinol (m.p. 120°C). This isomer is also predominant in the more volatile fractions. The different fractions were obtained as white solids (15.1 g, 75%).

### 3-Chloro-2,5,6-trifluoropyrid-4-yl acrylate (acrylic ester 10, AE10)

2,6-Lutidine (4.8g, 44.6 mmole) was added dropwise at room temperature to a stirred solution of 3-chloro-4-hydroxytrifluoropyridine (5.5 g, 30.0 mmole) in ether (100 ml) containing DMAP (catalytic). Stirring for 15 min was followed by dropwise addition of acryloyl chloride (5.5 g, 60.3 mmole). The reaction mixture was stirred vigorously for 45 minutes, then the precipitate was filtered and washed twice with ether.
The filtrates were concentrated and purification of the residue by elution through Florisil® (eluant: pentane/ dichloromethane 4/1) yielded acrylic ester 10 as a colorless oil (6.4 g, 90%).

### 3-Chloro-2,5,6-trifluoropyrid-4-yl, 5-chloro-3,4,6-trifluoropyrid-2-yl, 3-chloro-4,5,6-trifluoropyrid-2-yl, and 4-chloro-3,5,6-trifluoropyrid-2-yl acrylates (acrylic esters 10, 11, 12, and 13, AE10, AE11, AE12, AE13)

The procedure is the same as above, with the following materials a mixture of the pyridinols 3-chloro-4-hydroxytrifluoropyridine, 5-chloro-2-hydroxytrifluoropyridine, 3-chloro-2-hydroxytrifluoropyridine, and 4-chloro-2-hydroxytrifluoropyridine, with the respective percentages 60, 23, 3, and 14% (5.4 g, 29.4 mmole), 2,6-lutidine (4.7 g, 43.8 mmole), acryloyl chloride (5.3 g, 59.1 mmole) in ether (150 ml). Purification over Florisil® (eluant pentane/dichloromethane 3/2) yielded a mixture of isomeric acrylates as a colorless oil (6.7 g, 95%). The ¹⁹F-NMR spectrum showed the composition of this mixture to be 3-chloro-2,5,6-trifluoropyridin-4-yl acrylate (59%), 5-chloro-3,4,6-trifluoropyridin-2-yl acrylate (22%), 3-chloro-4,5,6-trifluoropyridin-2-yl acrylate (4%), and 4-chloro-3,5,6-trifluoropyridin-2-yl acrylate (15%).

### Examples 14, 15 and 16

### 4-Chlorotetrafluorophenol, 2-chlorotetrafluorophenol, and 3-chlorotetrafluorophenol.

A mixture of chloropentafluorobenzene (10 g, 49.4 mmole) and potassium hydroxide (8.3 g, 148.2 mmole) in tert-butanol (60 ml) was heated at 85°C for 30 h. After allowing the reaction medium to cool to room temperature, 70% of the solvent was evaporated under reduced pressure, and water was added until complete dissolution of the residue. This aqueous phase was acidified with 5N hydrochloric acid, the lower phase was decanted, and the upper phase was extracted with diethylether. The oil and the ethereal phases were combined, washed by water and dried over MgSO₄. After evaporating ether under reduced pressure, the residue was distilled under vacuum (54-56°C/3-4 mm Hg), giving a mixture of chlorotetrafluorophenols (7.6 g, 77%) as a bluish oil. The ¹⁹F-NMR spectrum showed the composition of this mixture to be 4-chlorotetrafluorophenol (72%), 2-chlorotetrafluorophenol (21%), and 3-chlorotetrafluorophenol (7%).

### 4-Chlorotetrafluorophenyl, 2-chlorotetrafluorophenyl, and 3-chlorotetrafluorophenyl acrylates (acrylic esters 14, 15, and 16, AE14, AE15, AE16)

First 2,6-lutidine (5.2 g, 48.9 mmole), then acryloyl chloride (4.4 g, 48.6 mmole) were added dropwise to a stirred solution of the preceding mixture of chlorotetrafluorophenols in THF (50 ml), cooled at 0°C, and containing DMAP (catalytic). After stirring for 45 minutes, the precipitate was filtered and washed twice with pentane. The filtrates were concentrated and purification of the residue by elution through Florisil® (eluant : pentane/dichloromethane 4/1) afforded a mixture of chlorotetrafluorophenyl acrylates as a colorless oil (7.1 g, 86%). The ¹⁹F-NMR spectrum showed the composition of this mixture to be 4-chlorotetrafluorophenyl acrylate (73%), 2-chlorotetrafluorophenyl acrylate (21%), and 3- chlorotetrafluorophenyl acrylate (6%).

### Examples 17, 18, 19 and 20

### 2,4-Dichlorotrifluorophenol, 2,6-dichlorotrifluorophenol, 3,4-dichlorotrifluorophenol, and 2,3-dichlorotrifluorophenol

A reaction between a mixture of dichlorotrifluorobenzene (1,3-dichloro-, 1,2-dichloro-: 85:15, 2 g, 9.1 mmole) and potassium hydroxide (1.5 g, 27.4 mmole) in tert-butanol (7 ml) was carried out in the same way as for the synthesis of the chlorotetrafluorophenols of examples 14-16. Kugelrohr distillation of the crude product under vacuum (100°C, 3mm Hg), then short-path distillation under 0.05 mm Hg yielded a mixture of dichlorotrifluorophenols as a colorless oil (1.4 g, 69%). The ¹⁹F-NMR spectrum showed the composition of this mixture to be 2,4-dichlorotrifluorophenol (80%), 2,6-dichlorotrifluorophenol (6%), 3,4-dichlorotrifluorophenol (11%), and 2,3-dichlorotrifluorophenol (3%).

### 2,4-Dichlorotrifluorophenyl, 2,6-dichlorotrifluorophenyl, 3,4-dichlorotrifluorophenyl, and 2,3-dichlorotrifluorophenyl acrylates (acrylic esters 17, 18, 19, and 20, AE17, AE18, AE19, AE20).

The procedure is the same as for the acrylic esters 14-16, with the following materials: the preceding mixture of dichlorotrifluorophenols 17-20 (2.3 g, 10.6 mmole), 2,6-lutidine (1.4 g, 16.0 mmole), acryloyl chloride (1.7 g, 16.3 mmole) in THF (25 ml). The reaction mixture was stirred for 20 minutes and purification over Florisil (eluant pentane/dichloromethane 3/2) yielded a mixture of dichlorotrifluorophenyl acrylates as a colorless oil (2.0 g, 69%). The 19F-NMR spectrum showed the composition of this mixture to be 2,4-dichlorotrifluorophenyl acrylate (80%), 2,6-dichlorotrifluorophenyl acrylate (6%), 3,4-dichlorotrifluorophenyl acrylate (11%), and 2,3-trichlorotrifluorophenyl acrylate (3%).

### Example 21

### Preparation of the polymers

Polymers were obtained by polymerizing the acrylic esters according to the invention by photochemical curing (HPK 125 W Hg-lamp, using optical filters to filter out the wavelengths below 360 nm) using Darocur 1173 (1 wt%), ex Ciba Geigy, as a photoinitiator. In some cases, especially when methacrylates were present in the monomer mixture, the polymers were submitted to a thermal post-curing treatment using azobis(isobutyronitril) (AIBN) as a thermal initiator. Post-curing was done for 1 hour at 70-80°C. To improve the mechanical and thermal stability ethyleneglycol di(meth)acrylate (EGD(M)A), triallylcyanurate (TAC) or butane do diacrylate (BDA) were added as a cross-linker.

The Tgs (measured with DSC second heating run, unless indicated otherwise) of the polymers (all UV and post-cured) are given in TABLE I.

**Table I**

| Polymer No. | Monomer in the monomer mixture | Tg (°C) |
|---|---|---|
| P1 | AE1 | 60-69 |
| P2 | AE2 | 120-134 |
| P3 | AE1/TeCEA*/EGDMA (32:62:5 wt%) | - |
| P4 | AE2/TeCEA*/EGDMA (49.5:44.5:5 wt%) | - |
| P5 | AE1/TeCEA/AIBN (27:72:0.15 wt%) | 68-75 |
| P6 | AE2/TeCEA*/EGDMA/AIBN 24.4:69.5:5:0.1 wt%) | 74-88 |
| P7 | AE2/TeCEA*/AIBN (44.9:54:0.1 wt%) | 76-100 |
| P8 | AE2/TeCEA*/EGDMA/AIBN (42.4:51.5:5:0.1 wt%) | 95-116 |
| P9 | AE14/AE15/AE16/AIBN (73:21:6:0.1 wt%) | 61-72 |
| P10 | AE17/AE18/AE19/AE20/AIBN (80:6:11:3:0.1wt%) | 66-81 |
| P11 | AE10/AIBN (99.9:0.1 wt%) | 74-86 |
| P12 | AE10/Ae11/AE12/AE13/AIBN (59:22:4:0.1 wt%) | 69-77 |
| P13 | AE1/TeCEA* (27.5:71.5 wt%) | 54-64 |
| P14 | AE1/TeCEA*/TAC (26.5:70.5:2 wt%) | 69-77 |
| P15 | AE1/TeCEA*/TAC/AIBN (25.85:71:2:0.15 wt%) | 71-80 |
| P16 | AE1/TeCEA*/TAC/AIBN (24.35:69.5:5:0.15 wt%) | 70-85 |
| P17 | AE1/TeCEA*/BDA/AIBN (25.85:71:2:0.15 wt%) | 68-77 |
| P18 | AE1/TeCEA*/BDA/AIBN (24.35:69.5:5:0.15 wt%) | 72-81 |
| P19 | AE2/TeCEA*/AIBN (44.85:54:0.15 wt%) | 77-95 |
| P20 | AE2/TeCEA*/TAC/AIBN (43.85:53:2:0.15 wt%) | 91-104 |
| P21 | AE2/TeCEA*/TAC/AIBN (42.35:51.5:5:0.15 wt%) | 89-103 |
| P22 | AE2/TeCEA*/BDA/AIBN (43.85:53:2:0.15 wt%) | 89-103 |
| P23 | AE2/TeCEA*/BDA/AIBN (42.35:51.5:5:0.15 wt%) | 90-101 |
| P24 | AE1/TeCEA*/AIBN (26.9:72:0.1 wt%) | 68-76 |
| P25 | AE1/TeCEA*/EGDMA/AIBN (25.9:71:2:0.1 wt%) | - |
| P26 | AE1/TeCEA*/EGDA/AIBN (25.6:70.7:2:0.1 wt%) | - |
| P27 | AE1/TeCEA/EGDA/AIBN (23.1:69.2:5:0.1 wt%) | 74-83 |
| P28 | AE2/TeCEA/EGDMA/AIBN (43.9:53:2:0.1 wt%) | - |
| P29 | AE2/TeCEA*/EGDA/AIBN (43.6:52.7:2:0.1 wt%) | - |
| P30 | AE2/TeCEA*/EGDA/AIBN (41.65:50.7:5:0.1 wt%) | 91-102 |
| P31 | AE5/AIBN (99.9:0.1 wt%) | 75-82 |

| | | |
|---|---|---|
| *) TeCEA = tetrachloroethylacrylate | | |

The intrinsic light losses of the polymers were measured according to the cutback method. In the cutback method a polymer block or polymer waveguide is prepared and light is guided through said film or waveguide. The light intensities of the light coupled into the block or waveguide (Iᵢₙ) and the light coupled out of the film (Iₒᵤₜ) is measured. Part of the block or waveguide is cut off and the Iᵢₙ and Iₒᵤₜ are measured again. In this way loss in dB/cm can be calculated. The results are compiled in TABLE II.

**Table II**

| Polymer No. | loss at 1300 nm (dB/cm) | loss at 1550 nm (dB/cm) |
|---|---|---|
| PMMA | 0.3 | 0.8 |
| P1 | <0.1 | <0.2 |
| P2 | 0.1 | 0.3 |
| P3 | 0.1 | 0.2 |
| P4 | 0.1 | 0.3 |

### Example 22

The maximal absorption of the polymers in the wavelength range of 1200-1600 nm was measured by means of a spectrophotometer. All polymers (P1-P31) appeared to have maximum light loss of lower than 1.5 dB/cm. P1 appeared to have a maximum light loss of lower than 0.9 dB/cm. PMMA appeared to have a maximum light loss of 5 dB/cm in the wavelength range of 1200-1600 nm.

The Indices of refraction (at 21°C) of the polymers obtained, measured with an Abbe refractometer or in a photogoniometer, are compiled in Table III.

**Table III**

| Polymer No. | n (at 633 nm) | n (at 830 nm) | n (at 1300 nm) | n (at 1550 nm) |
|---|---|---|---|---|
| PMMA | 1.490 | 1.484 | 1.481 | 1.479 |
| P1 | 1.477 | | 1.465 | 1.463 |
| P2 | 1.486 | | 1.478 | 1.477 |
| P3 | 1.510 | 1.504 | 1.500 | 1.498 |
| P4 | 1.503 | 1.497 | 1.493 | 1.491 |
| P10 | 1.537 | 1.526 | 1.513 | 1.513 |
| P12 | 1.525 | 1.513 | 1.510 | 1.508 |
| P31 | 1.492 | 1.484 | 1.477 | 1.474 |

### Example 23

With the acrylate ester mixture obtained in Examples 14, 15, 16 copolymers were obtained using different amounts of EGDMA (0.5, 0.9 and 3.3 wt%, respectively), to obtain polymers P32, P33, and P34. The various indices of refraction are compiled in TABLE IV.

**TABLE IV**

| Polymer No. | n (at 633 nm) | n (at 830 nm) | n (at 1300 nm) | n (at 1500 nm) |
|---|---|---|---|---|
| P32 | 1.509 | 1.500 | 1.494 | 1.493 |
| P33 | 1.509 | 1.501 | 1.495 | 1.493 |
| p34 | 1.510 | 1.502 | 1.495 | 1.495 |

### Example 24

Copolymers were prepared from the acrylate ester mixture obtained in examples 14, 15 and 16 with AE1 (72.5, 49.7 and 24.9 wt%, respectively) to obtain polymers P34, P35, and P36. The indices of refraction are compiled in TABLE V.

**TABLE V**

| Polymer No. | n (at 630 nm) | n (at 830 nm) | n (at 1300 nm) | n (at 1550 nm) |
|---|---|---|---|---|
| P1 | 1.478 | 1.471 | 1.467 | 1.465 |
| P35 | 1.484 | 1.478 | 1.475 | 1.471 |
| P36 | 1.493 | 1.487 | 1.481 | 1.482 |
| P37 | 1.499 | 1.493 | 1.486 | 1.487 |
| P9 | 1.507 | 1.499 | 1.494 | 1.493 |

### Example 25

### Preparation of polymers of pentafluorophenyl acrylate

In a typical experiment 3.48 g of pentafluorophenyl acrylate (FW = 252.15, 13.8 mmoles), 12.2 mg of AIBN (2,2'-azobisisobutyronitrile, FW = 164.21, 0.074 mmoles) were dissolved in 17.3 ml of 2-butanone (methyl ethyl ketone) in a 50 ml three-necked flask, provided with a magnetic stirring bar, vacuum /nitrogen line, and an oil bath.
After careful degassing of the reaction mixture to expell oxygen, the polymerisation was started by heating and stirring the flask's contents to circa 60°C under the exclusion of atmospheric oxygen.

After 20 hours, the polymerisation was discontinued, the solvent was almost completely removed by means of a rotary evaporator, the residue was redissolved in 6.5 ml of tetrahydrofurane, and the dissolved polymer was recovered through precipitation in methanol (75 ml) and filtration through a G4 glass filter. After drying in order to remove residual methanol/tetrahydrofurane (20 hours, 40°C, in vacuo), 2.3 g of poly(pentafluorophenyl)acrylate were obtained (yield circa 66%) (Mw = 35,800 ; Mn = 13,415, polydispersity 2.7).

Additional experiments yielded polymers with the following characteristics :
Mw = 10,700 ; Mn = 7,200, polydispersity 1.5;
Mw = 22,500 ; Mn = 9,300, polydispersity 2.4 respectively,
   depending on the experimental conditions applied, viz. the concentration of reactants, the reaction time, and the temperature of polymerisation, which is well known to those skilled in the art.

## Claims

1. A process for the preparation of (meth)acrylates of halogenated alcohols by direct esterification of said alcohols with (meth)acrylic acid or derivatives thereof wherein at least a 2,6-substituted pyridine derivative is used as a polymerization inhibiting catalyst at a reaction temperature of lower than 50°C.

2. The process according to claim 1 wherein the reaction temperature is at or below room temperature.

3. The process according to claim 1 or 2 wherein acrylates of halogenated alcohols are obtained by direct esterification of said alcohols with acrylic acid or derivatives thereof.

4. The process according to any one of claims 1-3 wherein the halogenated alcohol is chosen from any one of formulae 1-15: 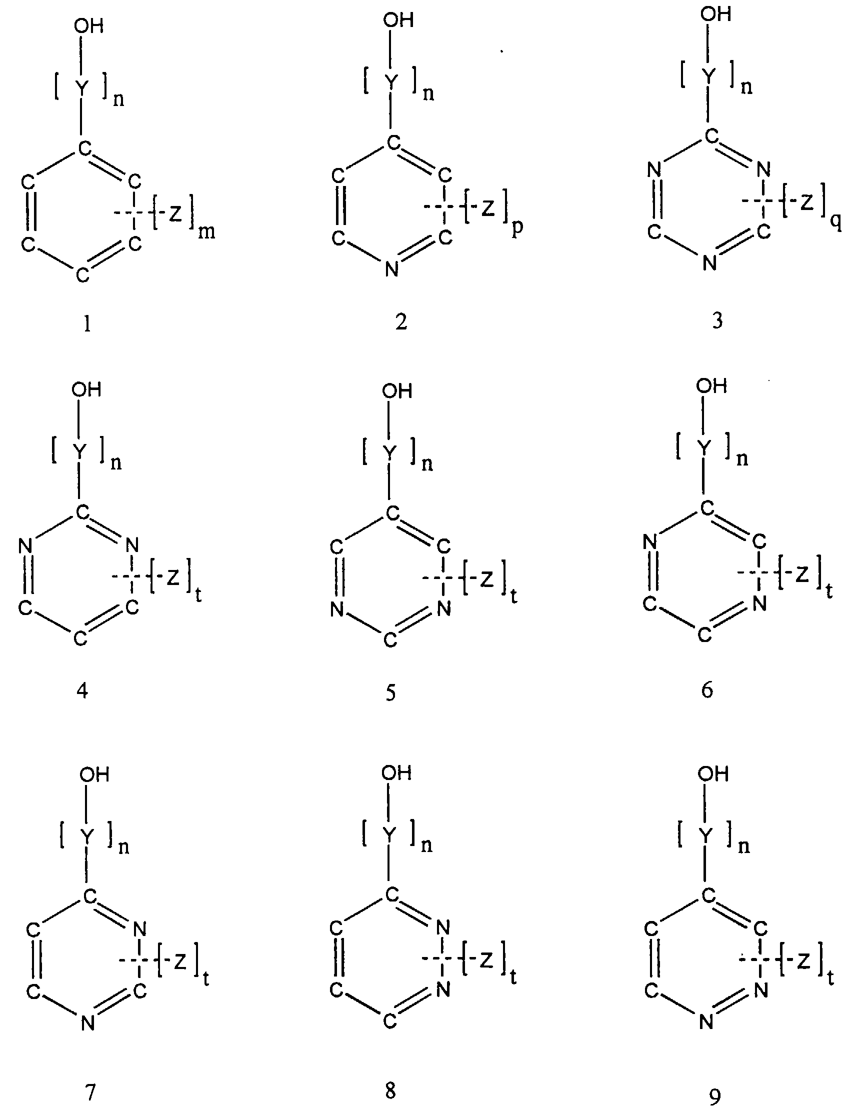 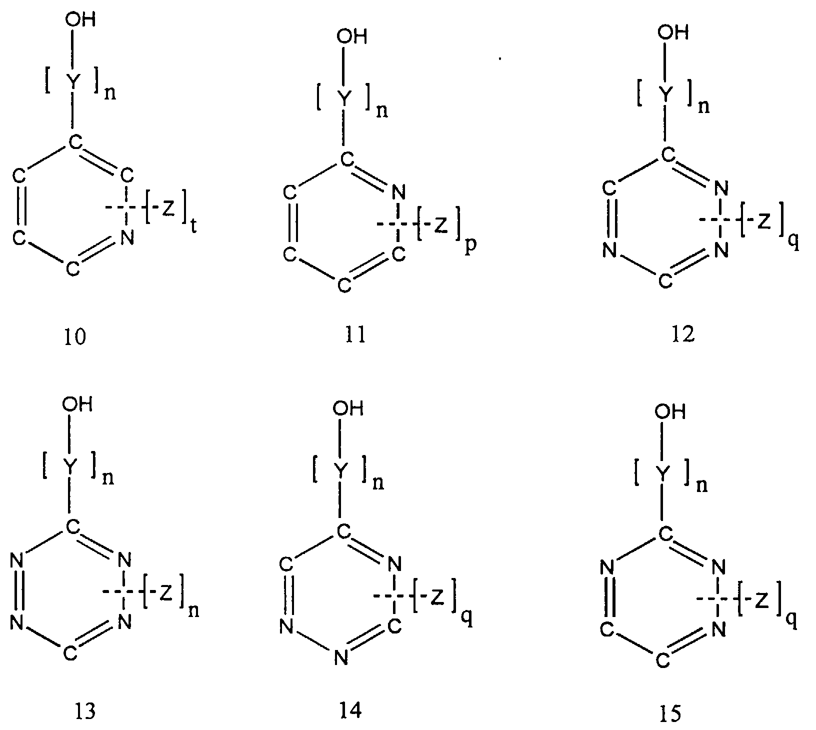 wherein
Y stands for -C(Chal₃)₂-,
n stands for 1 or 0,
hal stands for halogen,
Z stands for -F, -Cl, -D, -Br, or -CF₃,
m is an integer 1-5, wherein the Z atoms can be chosen independently from each other,
p is an integer 1-4, wherein the Z atoms can be chosen independently from each other,
q is an integer 1-2, wherein the Z atoms can be chosen independently from each other,
t is an integer 1-3, wherein the Z atoms can be chosen independently from each other.

5. The process according to any one of claims 1-4 wherein the (meth)acrylic acid derivative is selected from one of the following formulae: 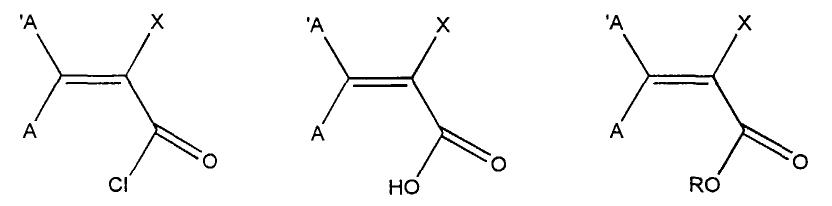 wherein
X stands for -H, -CH₃, -F, -Cl, -OCF₃, -OCCl₂F, -OCClF₂, -SCF_{3,} -SCCl₂F, -SCClF₂,wholly or partially halogenated linear alkyl group having 1-6 carbon atoms, a wholly or partially halogenated branched alkyl group having 1-6 carbon atoms,
A stands for -H, -D, or -hal,
A 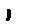 is independently chosen from A and may be the same groups as A.
R stands for an alkyl group having 1-6 carbon atoms.

6. The process according to any one of claims 1-5 wherein the at least 2,6-substituted pyridine derivative is 2,6-lutidine, 2,4,6-collidine, 2,6-di-tert.butylpyridine or 2,6-di-tert.butyl-4-methyl pyridine.

7. A (meth)acrylates of a halogenated alcohol obtainable by the esterification of alcohols according to formulae 2-15 and (meth)acrylic acid.

8. A homo- or copolymer obtainable by the polymerization of a monomer mixture comprising the (meth)acrylates of halogenated alcohols prepared by the process according any one of claims 1-6.

9. The copolymer of claim 8 wherein the monomer mixture comprises pentafluorophenyl (meth)acrylate and tetrachloroethyl acrylate.

10. The copolymer of claim 9 wherein the monomer mixture comprises 35-55 wt% pentafluorophenyl acrylate and 45-65 wt% tetrachloroethyl acrylate.

11. The copolymer of claim 10 wherein the monomer mixture comprises 45-60 wt% pentafluorophenyl methacrylate and 50-35 wt% tetrachloroethyl acrylate.

12. The copolymer of claim 10 or 11 wherein the monomer mixture further comprises upto 10 wt% non-halogenated cross-linker.

13. A waveguide structure, characterized in that it comprises the homo- or copolymer according to any one of claims 8-12.

14. The waveguide structure of claim 13 wherein the homo- or copolymer is used as core material, as cladding, or both as core material and cladding.

15. The waveguiding structure according to claim 13 or 14 wherein the cladding is composed of a moulded article with a recess, in which recess core material is provided.
